# EUROPEAN PATENT APPLICATION

(11) **EP 3 196 854 A1**
(43) Date of publication of application: **26.07.2017**
(21) Application number: 16305053.7
(22) Date of filing: 21.01.2016
(51) Int. Cl.: G08B 21/04, G06Q 50/22, G08B 29/18, G08B 21/02, G01S 11/06

(54) **INDOOR ACTIVITY DETECTION BASED ON TAG TRACKING**

(71) Applicant: Thomson Licensing, 92130 Issy-les-Moulineaux (FR)
(72) Inventor: GILBERTON, Philippe, 35576 Cesson-Sévigné (FR); LOUZIR, Ali, 35576 Cesson-Sévigné (FR); HOWSON, Christopher, 35576 Cesson-Sévigné (FR)
(74) Representative: Huchet, Anne

(57) **Abstract**

A method and apparatus for detecting and reporting anomalous behavior of a user in an environment are described, by which the location of an RF tag worn by a user is estimated by one or more fixed RF readers based on received signal strength and timestamp information from the RF tag. An activy profile is derived from location data.

Initially collected activity profile data is used to learn and characterise a user's daily profile. This serves as reference for comparing with current activity profiles determined in the same way, in order to detect anmalous behaviour based on the result of the comparison.

## Description

### FIELD

The proposed method and apparatus relates to the detection of abnormal activities of elderly and handicapped people residing in their homes or non-medical residences.

### BACKGROUND

This section is intended to introduce the reader to various aspects of art, which may be related to the present embodiments that are described below. This discussion is believed to be helpful in providing the reader with background information to facilitate a better understanding of the various aspects of the present disclosure. Accordingly, it should be understood that these statements are to be read in this light.

Tracking indoor activity is becoming more and more relevant as data about people is dramatically increasing thanks to the IoT (Internet of Things) and connected sensors. Tracking activity results in pushing industries and academics into finding new ways to better exploit the tracking data and provide better and more relevant assistance and recommendations to the end user. An emerging use case that illustrates this fact is elderly care or handicapped health care service that targets millions of people worldwide that require some assistance especially as they become older and live alone. Such a new service addresses people that are in good health and plan and/or wish to remain in their own homes or in a dedicated elderly (senior) center but that need occasional assistance from medical staff or simply a visit from their relatives when some abnormal behavior occurs.

In the domain of the care of elderly and handicapped people staying in their own homes or in non-medical residences (such as senior apartment complexes or assisted living facilities), one key area of interest is the life habits of the individual. Care workers (care givers) monitoring the elderly or handicapped individuals may effectively use a report on the life patterns of the person as a tool for diagnosis of behavioral or health problems. Anomalies may be detected for further investigation and alerts may be raised for immediate action in the case of an emergency.

To monitor the functional health of a smart home resident and detect anomalous behavior, some solutions rely on automatic recognition of daily living activities like standing, sitting, sleeping, etc... This approach requires a data collection infrastructure in the environment (e.g., home) implementing generally a large number of sensors (bed and chair occupancy sensors, etc...), which can be complex and expensive to implement. In addition such sensors (cameras, microphones, etc...) may sometimes be considered to be intrusive by the individual, consequently not widely accepted by elderly or handicapped individuals.

Other nonintrusive anomaly detection approaches rely on sensors located in the living environment such as motion detectors or door contactors, enabling the individual not to be disturbed with the technology.

One of the key questions that such a system needs to answer when such a system monitors daily activity of an elderly person (user) is: is the elderly daily activity today abnormal or not? This implies a system incorporating methods for learning activity habits of the monitored person over time, detecting anomalies and raising alerts. Furthermore the system must be unobtrusive and acceptable for an elderly person to wear. If the system is not unobtrusive then the tracking devices are not likely to be used.

One way of characterizing the behavior, or the activity, of the monitored individual (person, user) is to obtain some sort of measurement of his/her position over time. Accurate indoor localization is known to be a difficult and potentially costly problem to solve. On the other hand, simple proximity detection approaches are low cost but lacking in positional accuracy. Known proximity detection solutions from state of the art are based on the tag (beacon) paradigm meaning a tiny device is able to wirelessly transmit a unique identification in such a way that another device named listener or reader is able to detect the beacon of the signal emitting device in a limited range. Common RF (Radio Frequency) based solutions are using BLE (Bluetooth Low Energy) technology including Apple's iBeacon approach. Some commercialized examples: http://bealder.com/ is an application that tracks proximity of people around retail centers or malls; http://www.kensington.com/en/gb/4570/kensington#.VYkIYWcw-Uk is used for retrieving or keeping your favorite objects; and http://www.9solutions.com/en/technology/ipcs-technology-breakdown is dedicated to indoor localization of assets, patient or medical staff within a medical care center. Another really compact, lightweight, low cost and power efficient approach is the RFID (Radio Frequency IDentification) technology that is used as a tag for tracking assets, for example, http://www.radiantrfid.com/virtual-asset-tracker.html.

The existing solutions addressing the business-to-business (B2B) market need a dedicated infrastructure to sense or localize the assets and those addressing the consumer market require the end user to keep his smartphone on him with Bluetooth enabled. The resulting effect is that there are too many constraints that are not transposable to the environment used for tracking daily activity of elderly or handicapped individuals.

### SUMMARY

The proposed method and apparatus is directed to tracking (monitoring) the indoor activity of a person (user) by means of a small, lightweight, wireless, wearable tag. The proposed method and apparatus is directed to indoor tracking of daily activity of people over time. It does not require determining an accurate position and it also minimizes the infrastructure and the end user constraints. That is, the monitoring equipment can be less complex. Both the wearable tag and receivers can be simpler and, therefore less costly. That being said, the proposed method and apparatus could work with more complex and expensive legacy equipment. The primary targeted application is elderly and handicapped care but the invention could be used in any system where an unobtrusive and cost effective solution for monitoring and characterizing the indoor physical activity of persons without the need of precise tracking system, is expected. While principally directed to detecting anomalous behavior of elderly or handicapped individuals, the proposed method and apparatus are not so limited and may be employed to monitor prisoners, children or other individuals that find themselves in restricted environments such as, but not limited to prisons, detention centers and or situations of house arrest. The description of the proposed method and apparatus uses elderly or handicapped individuals as examples. In order to be accepted by elderly and handicapped individuals and not disturb them, the data collection system must be as non-intrusive as possible.

There are many ways to address such a requirement ranging from systems using additional sensors to other ones using existing means already in the house. To reduce the scope of the problem, the proposed method and apparatus addresses systems that use a dedicated wearable sensor connected wirelessly to a box integrating a listener/reader function. However, for acceptance of such a wearable tracking system by the elderly, the user friendliness has to be carefully considered by the solution providers. To respect this paradigm, the tracking device which the elderly will wear on a daily basis has to be light, small, self-powered and wirelessly connected to collect the necessary data.

The proposed method and apparatus tracks the daily activity of at least one person within the home (restricted environment) based on RF wireless technology in a small form factor, self-powered, wearable tag associated with one or more listeners (listening devices). The principle of the activity monitoring (tracking) relies on collecting the RSSI (Receiver Signal Strength Indication) values throughout the day by means of the one or more listener(s) located in (a) suitable place(s) in the restricted environment (home, senior living center, school, prison, detention center etc.). While the proposed method and apparatus is described using RSSI values (data), the proposed method and apparatus may also use Received Channel Strength Indicator or any other data representative of received signal strength. The resulting captured data contributes to building a time profile of the monitored person's activity by providing the timestamped proximity to the listener on, for example, a daily time frame. By collecting such time profiles the typical behavior of the user can be learned over time and, by comparing new time profiles with learned habits, anomalous behavior can be detected.

A method and apparatus for creating an anomalous behavior detection log for reporting anomalous behavior of a user in an environment are described including logging, for an initial period of time, unique identification information for an RF tag, initial data representative of received signal strength and initial timestamp information from the RF tag by one or more fixed RF readers, the RF tag worn by the user, generating an initial activity profile of the user over the initial period of time, the initial activity profile based on an initial tag proximity estimation (tag proximity estimation refers to the proximity to the RF reader(s)). using the logged initial data representative of received signal strength and the initial timestamp information, creating a characterization of the user's life routine based on the user's initial activity profile, logging second data representative of received signal strength and second timestamp information from the RF tag by the one or more fixed RF readers, the RF tag worn by the user, generating a second activity profile of the user over a second period of time, the second activity profile based on a second tag proximity estimation (tag proximity estimation refers to the proximity to the RF reader(s)) using the logged second data representative of received signal strength and the second timestamp information, comparing the characterization of the user's life routines with the second activity profile and creating an anomalous behavior detection log by detecting anomalous behavior using the comparison.

### BRIEF DESCRIPTION OF THE DRAWINGS

The proposed method and apparatus is best understood from the following detailed description when read in conjunction with the accompanying drawings. The drawings include the following figures briefly described below:
Fig. 1 is a two dimensional view of an exemplary tag and receiver configuration.
Fig. 2 is a picture of an exemplary wearable MTag.
Fig. 3 is a graph of exemplary RSSI variation over time.
Fig. 4 is a graph of exemplary daily activities of two individuals each wearing an MTag.
Fig. 5 is a view of iso-RSSI values represented as circles around the RX.
Fig. 6 is a view of a home configuration having multiple receivers (RX units) to resolve the potential iso-RSSI issue.
Fig. 7 is a graph showing RSSI variation versus RX1 and RX2.
Fig. 8 is a graph showing the daily activity profiles of the same individual captured at two different months of the year.
Fig. 9 is a flowchart of an exemplary implementation of the proposed method.
Fig. 10 is a block diagram of an exemplary server or RGW that performs the proposed method of creating an anomalous behavior detection log for reporting anomalous behavior of a user in an environment.

It should be understood that the drawing(s) are for purposes of illustrating the concepts of the disclosure and is not necessarily the only possible configuration for illustrating the disclosure.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein represent conceptual views of illustrative circuitry embodying the principles of the disclosure. Similarly, it will be appreciated that any flow charts, flow diagrams, state transition diagrams, pseudocode, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, read only memory (ROM) for storing software, random access memory (RAM), and nonvolatile storage.

Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

In the claims hereof, any element expressed as a means for performing a specified function is intended to encompass any way of performing that function including, for example, a) a combination of circuit elements that performs that function or b) software in any form, including, therefore, firmware, microcode or the like, combined with appropriate circuitry for executing that software to perform the function. The disclosure as defined by such claims resides in the fact that the functionalities provided by the various recited means are combined and brought together in the manner which the claims call for. It is thus regarded that any means that can provide those functionalities are equivalent to those shown herein.

The proposed method and apparatus contributes to monitoring the daily activity of individuals (people, users) living in a restricted environment (house, senior living facility, school, prison, detention center etc.). The proposed method and apparatus targets more particularly elderly or handicapped care service for which getting relevant activity information during the day provides key markers to know (determine) if behavioral changes in the monitored individuals (users, persons) occur.

In order for such a method and system to be used, the system has to respect some constraints to be well accepted including to be user friendly, light weight, small and self-powered. To limit the scope of the technologies covered by such a monitoring system, the proposed method and apparatus uses RF wireless wearable devices that are able to send, on regular basis, a unique signal that a receiver is able to detect, store and process. As an example of such convenient technologies respecting those requirements could be BLE or RFID tags previously described above. The transmitters (e.g., MTags) and receivers (listeners) are not necessarily smart devices and may not have sufficient processing power to perform the proposed method, so the time-stamped event data may be transmitted to a gateway (e.g., residential gateway) or server for recordation (storage) and processing. The time-stamped event data collected for a first (initial) pre-determined period of time is to be used as a training set (training data, learning data). The training may be performed on data from one or from multiple receiving devices.

The learning may be supervised or unsupervised. In supervised learning, the training data is labeled by a person. In unsupervised learning, the training data is not labeled. The workflow of the proposed method and apparatus is supervised in the sense that the workflow involved a training phase to learn the classification of the observed behavior (habit). The training data required varies depending upon the type and duration of habit (behavior) to be learned (classified). For example, if the behavior (habit) is a daily habit (behavior) then at least two weeks of training data would be required (needed). If, however, the behavior (habit) is a monthly habit then at least two months of training data would be required (needed). Learning (training) may be accomplished by any means known in the art.

The data sent by the tag and collected by one or more receivers has to include at least one piece (unit) of information, which is the unique ID of the tag. As an example of such, the information could be the name of the people wearing the device or the remaining battery level of the tag or a unique identification number associated with the tag. The data collection periodicity is controlled by the receiver that estimates the RSSI (data representative of received signal strength) and adds a timestamp value to the estimated or measured RSSI (data representative of received signal strength). The value of the RSSI (data representative of received signal strength) varies according to the range/proximity of the tag worn by monitored (tracked) individuals to the receiver(s). In comparison to a service that requires the user to carry his/her smartphone and enable, for example, the Bluetooth interface, the proposed method and apparatus only requires the monitored individual to wear the BLE tag, which is much more convenient especially when elderly or handicapped care service is targeted. The form for such a tag can really be appealing, for example, the two wirelessly connected devices commercialized and shown at: https://www.bellabeat.com/ or http://ouraring.com/.

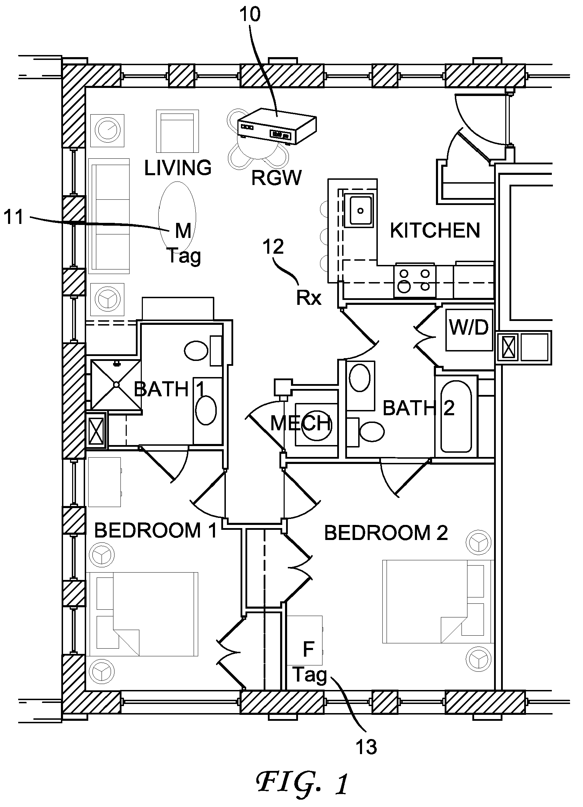

Fig. 1 shown an exemplary home architecture allowing deployment of such tracking activity sensing as follows:
RGW 10 is a Residential GateWay
Mtag 11 is a mobile tag worn by a monitored (tracked) individual
FTag 13 is an optional fixed position tag that allows performance of a calibration procedure explained further below
RX 12 is a receiver collecting the data from the tags (fixed or mobile) that is located in a suitable place of the house and that includes the necessary connection means to the RGW (Residential GateWay) to allow notification of an abnormal behavior alert/alaim to a dedicated external individual (medical staff, relatives, prison guards, school staff) through an internet connection (cellular network or similar). For privacy preservation of personal information, the data and possibly its processing could be performed locally and not in the cloud. The data storage and processing may also be performed in the RGW or a server.

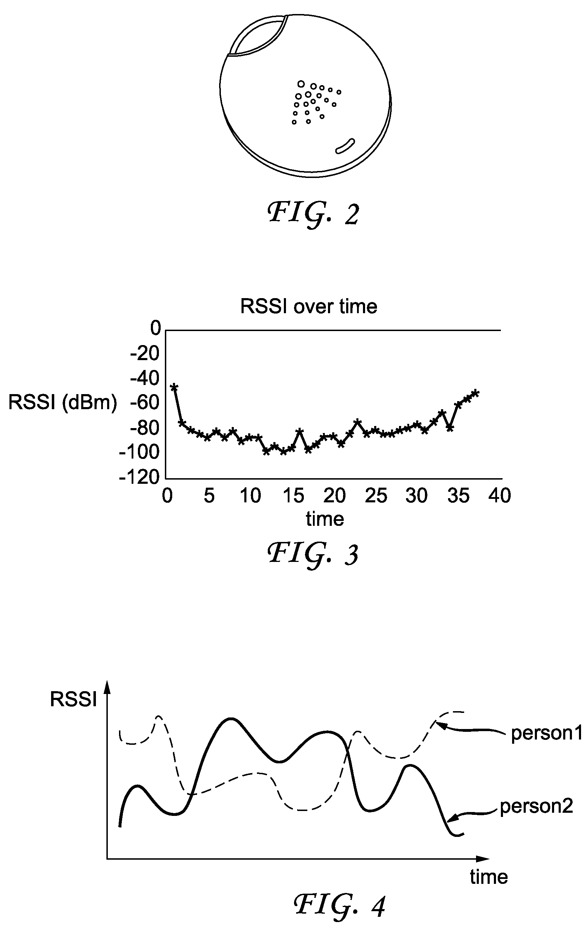

Fig. 2 shown an exemplary wearable tag (Mtag).

To better illustrate the benefit of using RSSI (data representative of received signal strength) values over time to track indoor activity of people, some results from an experiment are summarized hereafter:

The experiment was performed in a regular house using a smartphone (Samsung Galaxy S4) as an RX that collected the data (timestamped RSSI values), a mobile BLE tag as MTag that was worn by the monitored (tracked) person (individual, user). The person wearing the Mtag walked from the hall located at the 1^{st} floor close to where the RX was positioned. The monitored (tracked) individual then went upstairs and then went back to the hall close to the RX. The results are presented in Fig. 3 show clearly the RSSI variation (considered as the proximity variation) over time between the MTag and the RX. The data is clearly able to track the indoor daily activity of an individual. The MTag proximity monitoring will vary for the monitored (tracked) individual during the day.

Fig. 4 is a graph of exemplary daily activities of two individuals each wearing an MTag. Fig. 4 also shows that the proposed method and apparatus can be extended to monitor several individuals, each individual having a unique daily activity tracking profile. A daily activity tracking profile characterizes the activity of each person and it can be classified as regular activity (or abnormal activity) over different temporal windows: hourly, daily, weekly, seasonal, yearly, versus weekdays, versus weekend, etc...For example in identifying regular daily activity for an individual versus weekend activity with learning algorithms, it is possible to detect abnormal activity or trends over time that could be helpful for care givers or medical staff to be alerted about.

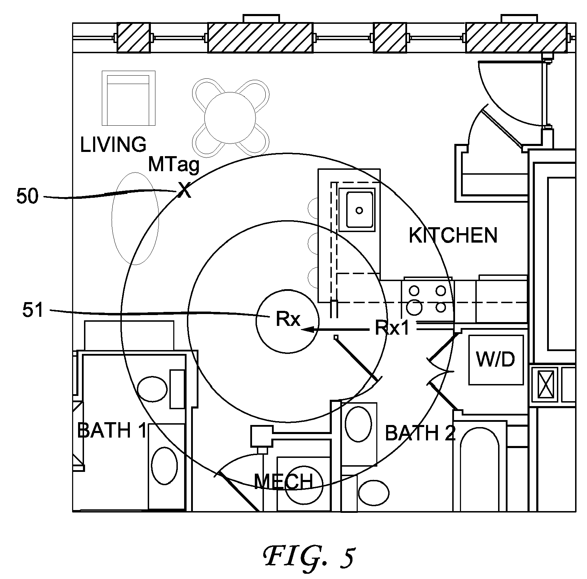

The proposed method and apparatus for collecting the RSSI (data representative of received signal strength) values over time works in most environments but there may be a problem in the situation where the monitored (tracked) individual walked around the RX 51 at an almost constant distance. Any reference to iso-RSSI values could as easily be any iso-data representative of received signal strength such as Received Channel Strength Indicator. Fig. 5 is a view of iso-RSSI values represented as circles around the RX. The iso-RSSI values of Fig. 5 are not unlike other iso-values, such as isobars. That is, the iso-values represent lines of equal distance from the RX in the case of iso-RSSI values. In the case of isobars, the lines represent lines of equal atmospheric pressure. In the situation represented in Fig. 5, the averaged RSSI (data representative of received signal strength) values would not vary enough to be representative of the daily activity of the monitored individual.

The three concentric circles in Fig. 5 are the iso-RSSI averaged values. That is, the receiver is recording (logging, collecting) a constant value as long as the monitored person is walking in an imaginary circle of a constant distance from the RX. So the RX will deduce that the monitored (tracked) individual is still and not walking at all. To mitigate this problem, another receiver (RX) located in a different place in the environment is used. The additional receiver allows the proposed method and apparatus to discriminate between no movement and movement at a constant distance from the first receiver.

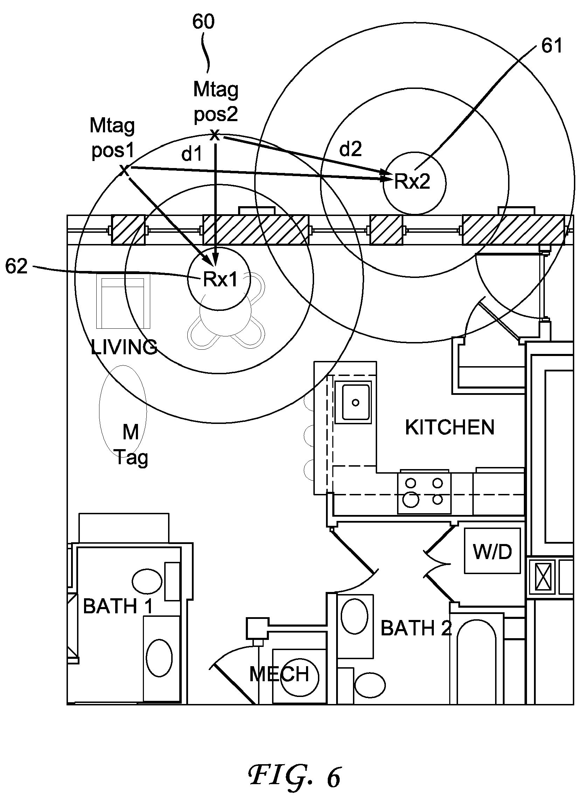

Fig. 6 is a view of a home configuration having multiple receivers (RX units) to resolve the potential iso-RSSI issue. In Fig. 6 RX1 62 and RX2 61 are now located in two different places in the environment (e.g., home, residence, senior living center, prison, school, etc.). For ease of deploying such a configuration, it can be imagined that RX1 is integrated in an all in one capable "smart object" like Cocoon (https://www.indiegogo.com/projects/cocoon-complete-home-security-from-one-device#/story) and RX2 is the smartphone of the monitored individual in the environment and that both of receivers are Bluetooth enabled and are able to exchange or send (transmit, forward) their collected RSSI values (data representative of received signal strength) to a common place for storage (recordation, logging) and further processing. Such a common place may be a gateway, RGW or server.

The location chosen for the placement of the second receiver should be far enough away from the first receiver and in a place so that it is likely that the RSSI values received by the two receivers are sufficiently different so that iso-data values representative of signal strength can be resolved (discriminated). At the same time, with such a choice, the system coverage could be improved. That is, the second receiver and any subsequent receivers could be used to better assess the user's position by the data provider or perhaps through triangulation as well as to disambiguate the iso-data issue.

In this configuration even when the MTag is moving on an imaginary track of a constant distance, RX1 and RX2 will receive different RSSI values (data representative of received signal strength) because the range d1 and d2 of the MTag is changing as between RX1 and RX2. The activity profile over time seen from the RX1 and RX2 could be as follows:

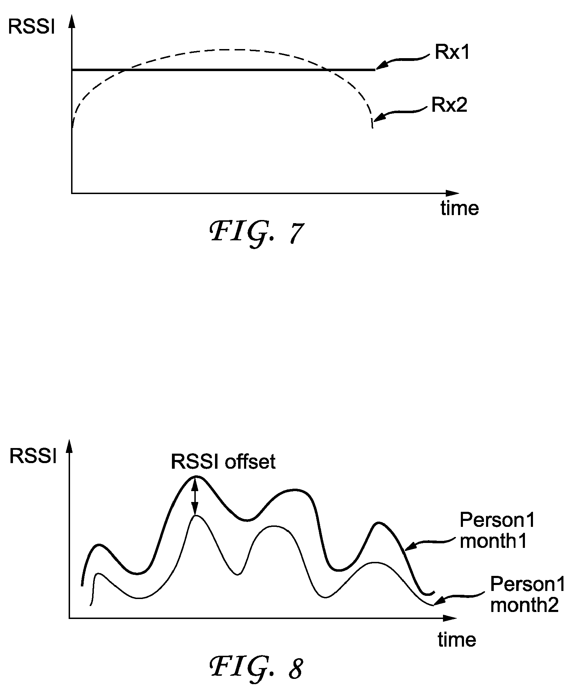

Fig. 7 is a graph showing RSSI (data representative of received signal strength) variation versus RX1 and RX2. The two curves superimposed in temporal x axis show that the monitored (tracked) individual is moving and not still.

In an alternative embodiment of the proposed method and apparatus smart object RX1 is replaced by a second smartphone assuming the two smartphones are no co-located in a common place in the environment but rather are located in two different rooms of the environment.

The fixed tag (FTag) allows for the calibration of the averaged RSSI values (data representative of received signal strength) measured by RX1 or RX2 by applying a RSSI offset value. This offset value could occur when the battery of the FTag is decreasing or when RSSI (data representative of received signal strength) fluctuation occurs. This radio frequency propagation phenomenon may occur over a long period of time such as a week, a month or a season. It means that in the same measurement configuration the averaged RSSI values (data representative of received signal strength) could be different of a few dB. In this case the FTag is used as an RSSI (data representative of received signal strength) reference and could be useful when the individual's daily activity profile needs to be compared for example on a monthly basis such as shown in Fig. 8, which is a graph showing the daily activity profiles of the same individual captured at two different months of the year.

Applying an offset will superimpose the curves and could help a decision engine to evaluate more accurately abnormal (anomalous) behavior and send more appropriate notifications to the care giver or the medical staff or the monitor. This calibration could be assimilated as a normalization process that is usually used when data needs to be compared.

This method is applicable to RFID technology or any similar RF technology that would be characterized by offering to use/wear a tiny, low cost and self-powered tagging device. RFID technology allows the use of passive, semi-active or active tags. The use of passive tags, while it is interesting from cost, power and user's acceptability point of view, its indoor read range could be limited to only a few meters. In this case several RFID readers connected in network (wired or wireless using ZigBee like technology) may be required to cover the entire environment.

The proposed method and apparatus is able to monitor (track) an individual's movements without the need for costly indoor positioning hardware. The proposed method and apparatus is light weight, user friendly and the monitored (tracked) individual does not need to carry a smartphone or similar device with them at all times but rather need only wear a non-intrusive wireless tagging device (Mtag) such as Bellabeat or Ouraring. The proposed method and apparatus has the ability to monitor multiple people in the environment as well as to calibrate the RSSI (data representative of received signal strength) values.

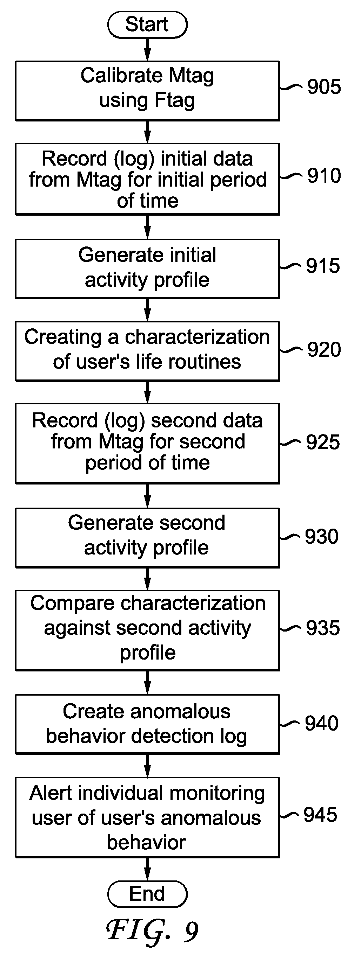

Fig. 9 is a flowchart of an exemplary implementation of the proposed method of creating an anomalous behavior detection log for reporting anomalous behavior of a user in an environment. At 905 the MTag worn by the monitored user is calibrated using the FTag. At 910 initial (first) data from the MTag worn by the monitored user is recorded (logged) for an initial (first) period of time. The data includes unique identification information of the RF tag (MTag) worn by the user, timestamp information from said RF tag by one or more fixed RF readers and RSSI data (data representative of received signal strength). The use of a second RF reader allows (permits) the disambiguation of iso-RSSI readings. At 915 an activity profile of the user is generated over the initial (first) period of time. The initial activity profile is based on an initial tag proximity estimation (tag proximity estimation refers to the proximity to the RF reader(s)) using the logged initial RSSI (data representative of received signal strength) data and the initial timestamp information. At 920 a characterization of the user's life routine is created based on the user's initial (first) activity profile. At 925 second RSSI (data representative of received signal strength) data and second timestamp information from the RF tag (worn by the user) is recorded (logged) by the one or more fixed RF readers. At 930 a second activity profile of the user over a second period of time is generated. The second activity profile is based on a second tag proximity estimation (tag proximity estimation refers to the proximity to the RF reader(s)) using the logged second RSSI data (data representative of received signal strength) and the second timestamp information. At 935 a comparison is made between the user's life routines with the second activity profile. At 940 an anomalous behavior detection log is created using results of the comparison. Any detected anomalous behavior in the anomalous behavior detection log is reported to the individual monitoring the user's activity at 945. The individual monitoring the user's activity may be a care giver, school staff, prison staff, a relative of said user or detention officer. The characterization of life routines is used as training data.

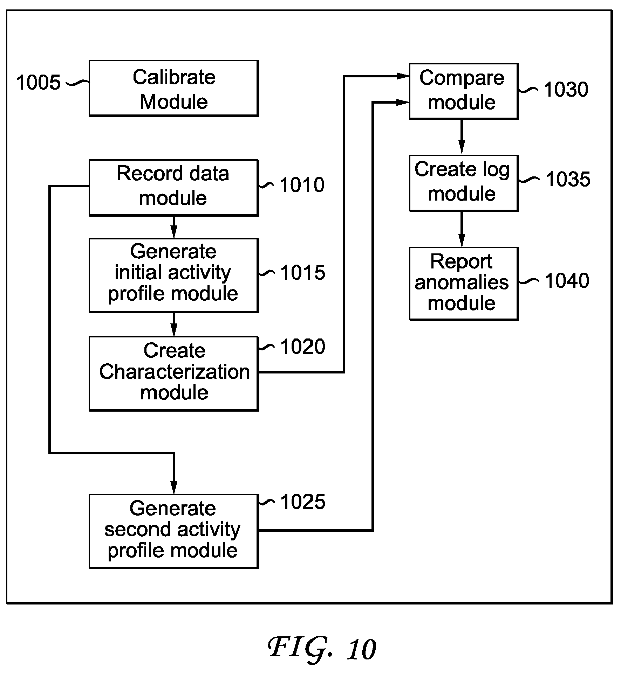

The proposed method may be performed in a residential gateway (RGW) or a server. Fig. 10 is a block diagram of an exemplary server or RGW that performs the proposed method of creating an anomalous behavior detection log for reporting anomalous behavior of a user in an environment. At 1005 the calibrate module calibrates the MTag worn by the monitored user using the FTag. Initial (first) data from the MTag worn by the monitored user is recorded (logged) for an initial (first) period of time by the record data module 1010. The data includes unique identification information of the RF tag (MTag) worn by the user, timestamp information from said RF tag by one or more fixed RF readers and RSSI data (data representative of received signal strength). An activity profile of the user is generated over the initial (first) period of time by the generate initial module 1015. The initial activity profile is based on an initial tag proximity estimation (tag proximity estimation refers to the proximity to the RF reader(s)) using the logged initial RSSI data (data representative of received signal strength) and the initial timestamp information. The use of a second RF reader allows (permits) the disambiguation of iso-RSSI readings. A characterization of the user's life routine is created based on the user's initial (first) activity profile by the create characterization module (1020). A second RSSI data (data representative of received signal strength) and second timestamp information from the RF tag (worn by the user) is recorded (logged) by the one or more fixed RF readers by the record data module (1010). A second activity profile of the user over a second period of time is generated by the generate second activity profile module (1025). The second activity profile is based on a second tag proximity estimation (tag proximity estimation refers to the proximity to the RF reader(s)) using the logged second RSSI data (data representative of received signal strength) and the second timestamp information. A comparison is made between the user's life routines with the second activity profile by the compare module (1030). An anomalous behavior detection log is created using results of the comparison by the create log module (1035). Any detected anomalous behavior in the anomalous behavior detection log is reported to the individual monitoring the user's activity by the report anomalies module (1040). The individual monitoring the user's activity may be a care giver, school staff, prison staff, a relative of said user or detention officer. The characterization of life routines is used as training data.

It is to be understood that the proposed method and apparatus may be implemented in various forms of hardware, software, firmware, special purpose processors, or a combination thereof. Special purpose processors may include application specific integrated circuits (ASICs), reduced instruction set computers (RISCs) and/or field programmable gate arrays (FPGAs). Preferably, the proposed method and apparatus is implemented as a combination of hardware and software. Moreover, the software is preferably implemented as an application program tangibly embodied on a program storage device. The application program may be uploaded to, and executed by, a machine comprising any suitable architecture. Preferably, the machine is implemented on a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof), which is executed via the operating system. In addition, various other peripheral devices may be connected to the computer platform such as an additional data storage device and a printing device.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces. Herein, the phrase "coupled" is defined to mean directly connected to or indirectly connected with through one or more intermediate components. Such intermediate components may include both hardware and software based components.

It is to be further understood that, because some of the constituent system components and method steps depicted in the accompanying figures are preferably implemented in software, the actual connections between the system components (or the process steps) may differ depending upon the manner in which the proposed method and apparatus is programmed. Given the teachings herein, one of ordinary skill in the related art will be able to contemplate these and similar implementations or configurations of the proposed method and apparatus.

## Claims

1. A method of creating an anomalous behavior detection log for reporting anomalous behavior of a user in an environment, said method comprising:
logging (910), for an initial period of time, unique identification information for an RF tag, initial data representative of received signal strength and initial timestamp information from said RF tag by one or more fixed RF readers, said RF tag being worn by said user;
generating (915) an initial activity profile of said user over said initial period of time, said initial activity profile based on an initial tag proximity estimation using said logged initial data representative of received signal strength and said initial timestamp information;
creating (920) a characterization of said user's life routine based on said user's initial activity profile;
logging (925) second data representative of received signal strength and second timestamp information from said RF tag by said one or more fixed RF readers, said RF tag worn by said user;
generating (930) a second activity profile of said user over a second period of time, said second activity profile being based on a second tag proximity estimation using said logged second data representative of received signal strength and said second timestamp information;
comparing (935) said characterization of said user's life routines with said second activity profile; and
creating (940) an anomalous behavior detection log by detecting anomalous behavior using said comparison.

2. The method according to claim 1, further comprising reporting any detected anomalous behavior of said user to a care giver, wherein said care giver is an individual monitoring said user's activity.

3. The method according to claim 1, wherein said characterization of life routines is used as training data.

4. The method according to claim 2, wherein said individual monitoring said user's activity is school staff, prison staff, a relative of said user or detention officer.

5. The method according to claim 1, further comprising calibrating said RF tag worn by said user using a fixed RF tag.

6. The method according to claim 1, wherein any RF readers beyond the first RF reader are used to better assess said user's position.

7. The method according to claim 1, wherein a second RF reader is used to disambiguate iso-data representative of received signal strength.

8. An apparatus for creating an anomalous behavior detection log for reporting anomalous behavior of a user in an environment, comprising:
means for logging (1010), for an initial period of time, unique identification information for an RF tag, initial data representative of received signal strength and initial timestamp information from said RF tag by one or more fixed RF readers, said RF tag being worn by said user;
means for generating (1015) an initial activity profile of said user over said initial period of time, said initial activity profile based on an initial tag proximity estimation using said logged initial data representative of received signal strength and said initial timestamp information;
means for creating (1020) a characterization of said user's life routine based on said user's initial activity profile;
means for logging (1010) second data representative of received signal strength and second timestamp information from said RF tag by said one or more fixed RF readers, said RF tag worn by said user;
means for generating (1025) a second activity profile of said user over a second period of time, said second activity profile being based on a second tag proximity estimation using said logged second data representative of received signal strength and said second timestamp information;
means for comparing (1030) said characterization of said user's life routines with said second activity profile; and
means for creating (1035) an anomalous behavior detection log by detecting anomalous behavior using said comparison.

9. The apparatus according to claim 1, further comprising reporting any detected anomalous behavior of said user to a care giver, wherein said care giver is an individual monitoring said user's activity.

10. The apparatus according to claim 1, wherein said characterization of life routines is used as training data.

11. The apparatus according to claim 2, wherein said individual monitoring said user's activity is school staff, prison staff, a relative of said user or detention officer.

12. The apparatus according to claim 1, further comprising means for calibrating said RF tag worn by said user using a fixed RF tag.

13. The apparatus according to claim 1, wherein any RF readers beyond the first RF reader are used to better assess said user's position.

14. The apparatus according to claim 1, wherein a second RF reader is used to disambiguate iso-data representative of received signal strength.
